# EUROPÄISCHE PATENTANMELDUNG

(11) **EP 4 147 691 A1**
(43) Veröffentlichungstag der Anmeldung: **15.03.2023**
(21) Anmeldenummer: 22193632.1
(22) Anmeldetag: 02.09.2022
(51) Int. Cl.: A61K 31/07, A61K 31/737, A61K 31/375, A61K 31/355, A61K 31/202, A23L 33/10, A23L 33/105, A23L 33/15, A23L 33/16, A61K 33/30, A61K 33/34, A61K 36/02, A61K 36/03, A61P 27/02

(54) **ZUSAMMENSETZUNG UND DARREICHUNGSFORM UMFASSEND ERSTES UND ZWEITES FUCOIDAN**

(30) Priorität: 10.09.2021 DE 202021104882 U
(71) Anmelder: Lighthouse Pharma GmbH, 81667 München (DE)
(72) Erfinder: POHL, Dr. Berthold, 82152 Krailling (DE)
(74) Vertreter: Zimmermann & Partner Patentanwälte mbB

(57) **Zusammenfassung**

Zusammensetzung, umfassend einen ersten Bestandteil, umfassend ein erstes Fucoidan, und einen zweiten Bestandteil, umfassend ein zweites Fucoidan, wobei sich der erste Bestandteil vom zweiten Bestandteil unterscheidet.

## Beschreibung

Die vorliegende Erfindung betrifft eine Zusammensetzung und eine Darreichungsform, umfassend ein erstes Fucoidan und ein zweites Fucoidan, insbesondere zur Verwendung als Nahrungsergänzungsmittel, ergänzende bilanzierte Diät oder zur Verwendung in der Behandlung und/oder Prävention von Erkrankungen der Netzhaut des Auges.

Fucoidan ist eine Klasse von natürlichen Verbindungen, die insbesondere in Braunalgen vorkommt. Fucoidan umfasst sulfatierte Polysaccharide basierend auf dem Zucker Fucose. Die molekulare Struktur des jeweiligen Fucoidans variiert in Abhängigkeit der Braunalgenspezies.

Braunalgen finden seit Langem Verwendung in der ostasiatischen Küche. Als Quellen von insbesondere mineralischen Mikronährstoffen werden Braunalgen als Nahrungsergänzungsmittel genutzt. Weitere bekannte Verwendungen von Braunalgen sowie der darin vorkommenden Fucoidane umfassen die Verbesserung der Darmgesundheit, sowie die Verwendung in Haarwuchspräparaten oder Wundauflagen. Für manche Fucoidane sind antioxidative, neuroprotektive, immunmodulierende und *vascular endothelial growth factor* (VEGF)-inhibierende Eigenschaften bekannt.

Die altersbedingte Makuladegeneration (AMD) ist eine der Hauptursachen für Erblindung bei über Fünfzigjährigen in Industriestaaten. Die AMD entsteht durch Alterung der Stützstrukturen der Retina, insbesondere dem retinalen Pigmentepithel, der Burch'schen Membran und der Aderhaut, durch eine Akkumulation von Lipofuszin in den Zellen des retinalen Pigmentepithels, die mit einer Unterversorgung des retinalen Pigmentepithels aufgrund einer Alterung der Aderhaut einhergehen kann. Eine Prävention und/oder Behandlung der AMD ist derzeit nicht in zufriedenstellender Weise möglich.

Es besteht somit Bedarf an einer Zusammensetzung und/oder einer Darreichungsform der Zusammensetzung, durch die der Eintritt und/oder der Verlauf der AMD zumindest teilweise verhindert oder verlangsamt werden kann, oder durch die eine AMD zumindest teilweise behandelt werden kann.

Die hierin beschriebenen Zusammensetzungen und Darreichungsformen lösen die genannten Probleme.

Gemäß einem ersten Aspekt wird eine Zusammensetzung beschrieben. Die Zusammensetzung umfasst einen ersten Bestandteil, umfassend ein erstes Fucoidan; sowie einen zweiten Bestandteil, umfassend ein zweites Fucoidan. Der erste Bestandteil unterscheidet sich vom zweiten Bestandteil.

Gemäß einem weiteren Aspekt wird eine Darreichungsform beschrieben. Die Darreichungsform umfasst eine Zusammensetzung nach einem der vorhergehenden Ansprüche. Die Darreichungsform kann insbesondere eine Darreichungsform zur peroralen Anwendung sein, insbesondere Tablette, Kapsel, Pulver, Granulat oder Liquid.

Gemäß einem weiteren Aspekt wird eine Zusammensetzung zur Verwendung bei der Behandlung und/oder Prävention der altersbedingten Makuladegeneration (AMD) bei Säugetieren beschrieben. Die Zusammensetzung umfasst eine erste Braunalgenspezies und/oder Derivate davon, umfassend ein erstes Fucoidan. Das erste Fucoidan weist eine antioxidative Eigenschaft auf. Die Zusammensetzung umfasst eine zweite Braunalgenspezies und/oder Derivate davon, umfassend ein zweites Fucoidan. Das zweite Fucoidan weist eine VEGF-hemmende Eigenschaft auf. Die Zusammensetzung umfasst ein Mikronährstoff-Gemisch, umfassend Vitamin C, Vitamin E, Zink, Kupfer, Lutein, und Zeaxanthin als oral bioverfügbare Bestandteile.

Gemäß einem Aspekt werden ein erster Bestandteil und ein zweiter Bestandteil einer Zusammensetzung beschrieben. Der jeweilige Bestandteil enthält ein erstes Fucoidan oder ein zweites Fucoidan. Der erste Bestandteil und der zweite Bestandteil können jeweils eine Algenspezies und/oder Derivate davon umfassen. In Ausführungsformen kann es sich bei dem Bestandteil um eine Präparation der Algenspezies handeln, insbesondere eine trockene oder getrocknete Präparation der Algenspezies, wobei hierin beschriebene Präparationen von Algenspezies der verbesserten Lesbarkeit wegen teilweise verkürzt als Algenspezies bezeichnet werden können. Die Algenspezies kann eine Braunalgenspezies sein.

Als Präparation einer Algenspezies ist jede Verarbeitungsform der Algenspezies zu verstehen. Die Präparation kann beispielsweise getrocknetes Pulver, Granulat, Flocken, Blätter und/oder Schnitte der Algenspezies umfassen, sowie Abwandlungen davon. Die Algenspezies kann beispielsweise geerntet, gewaschen, getrocknet und/oder in trockener Form weiterverarbeitet sein. In Ausführungsformen kann es sich bei dem Bestandteil um ein Derivat einer Algenspezies handeln, beispielsweise um behandelte Algen der Algenspezies, wie etwa gewaschene Algen, säurebehandelte Algen, sterilisierte, beispielsweise bestrahlte Algen, mechanisch verarbeitete, beispielsweise homogenisierte Algen, enzymatisch behandelte Algen, oder dergleichen.

Des Weiteren kann es sich bei dem Derivat um ein Extrakt einer Algenspezies handeln, wobei das Extrakt ein Fucoidan beinhaltet. Das Fucoidan kann zumindest teilweise abgebaut sein und/oder ein Abbauprodukt sein, beispielsweise ein enzymatisches Abbauprodukt oder ein Hydrolyseprodukt. Beispiele für bekannte Extraktionsmethoden sind in Lim et al., Seaweed Polysaccharides, 2017, 27-46, DOI: 10.1016/B978-0-12-809816-5.00003-7 genannt, wobei auch die darin genannten Extrakte, Isolate und Zwischenstufen als Derivate einer Algenspezies zu verstehen sind. Vorteilhafterweise kann ein Derivat einer Algenspezies in der Zusammensetzung in getrockneter Form, beispielsweise als Pulver, Granulat, Flocken, oder dergleichen vorliegen. In weiteren Ausführungsformen kann das Derivat der Algenspezies auch in flüssiger Form, beispielsweise als Gel, Suspension, Emulsion oder Lösung vorliegen.

Gemäß einem Aspekt wird ein erstes Fucoidan und ein zweites Fucoidan beschreiben. Das Fucoidan kann ein natürliches, von einer jeweiligen Algenspezies gebildetes Fucoidan sein. Das Fucoidan kann durch die Gewinnbarkeit aus einer Algenspezies definierbar sein. Das Fucoidan kann eine für aus natürlichen Quellen gewonnenen Stoffen typische Inhomogenität aufweisen. Das Fucoidan kann durch die Struktur des Kohlenhydrat-Backbones definierbar sein. Das Fucoidan kann durch spezifische Derivatisierungen, beispielsweise spezifische Sulfatierung oder Acetylierung, definierbar sein. Das Fucoidan kann durch einen Derivatisierungsgrad, beispielsweise die relative Anzahl an Sulfat- oder Acetylgruppen definierbar sein. Das Fucoidan kann durch die molekulare Größe definierbar sein. Das Fucoidan kann durch die relative Menge an gebundenen Spurenelementen, beispielsweise Iod, definierbar sein.

Die chemische und strukturelle Beschreibbarkeit von Fucoidanen bestimmer Algenspezies, insbesondere von Braunalgenspezies, ist bekannt. Beispielsweise wird das Fucoidan der Braunalge *Saccharina latissima* in Bilan et al., Carbohydrate Research, 2010, 345, 2038-2047, DOI: 10.1016/j.carres.2010.07.009 beschrieben. Beispielsweise wird das Fucoidan der Braunalge *Laminaria hyperborea* in Kopplin et al., ACS Appl. Bio Mater. 2018, 1, 6, 1880-1892, DOI: 10.1021/acsabm.8b00436 beschrieben.

Gemäß einem Aspekt weist das erste Fucoidan eine antioxidative Eigenschaft auf. Das erste Fucoidan kann ein Antioxidans mit einer antioxidativen Eigenschaft umfassen. Eine antioxidative Eigenschaft kann dann vorliegen, wenn das Fucoidan *in vitro* und/oder *in vivo,* insbesondere bei oraler Gabe, eine Minderung des oxidativen Stresses in einem Organismus bewirkt. Als Minderung des oxidativen Stresses kann eine zumindest zeitweise Verringerung der Menge oder Konzentration an reaktiven Sauerstoffverbindungen und/oder freien Radikalen bezeichnet werden. Im Sinne dieser Offenbarung gilt insbesondere ein in der Braunalge *Saccharina latissima* enthaltenes Fucoidan als ein Fucoidan mit einer antioxidativen Eigenschaft. Weitere Fucoidane mit antioxidativen Eigenschaften sind in Algen der Gattung *Fucus* enthalten, insbesondere *Fucus vesiculosus, Fucus Distichus subsp evanescens* oder *Fucus serratus.* Weitere Fucoidane mit antioxidativen Eigenschaften können ebenso in Spezies des Taxons *Laminariales* und/oder *Fucales* enthalten sein.

Gemäß einem Aspekt weist das zweite Fucoidan *in vitro* und/oder *in vivo* eine VEGF-hemmende Eigenschaft auf, insbesondere bei oraler Gabe. Als VEGF ist ein *vasuclar endothelial growth factor* zu verstehen. Das Fucoidan kann eine VEGF modulierende oder inhibierende Eigenschaft aufweisen. Das Fucoidan kann eine VEGF-hemmende Eigenschaft aufweisen, indem es VEGF, einen VEGF-Rezeptor und/oder einen VEGF-bindenden Rezeptor bindet, beispielsweise als Agonist, Modulator oder Inhibitor. Das Fucoidan kann eine VEGF-hemmende Eigenschaft aufweisen, indem es eine VEGF-Signalkaskade moduliert. Eine VEGF-hemmende Wirkung kann als eine Wirkung beschrieben werden, durch die die Gefäßbildung, Angiogenese und/oder die Angioneogenese zumindest teilweise unterdrückt oder gemindert wird. Im Sinne dieser Offenbarung gilt insbesondere ein in der Braunalge *Laminaria hyperborea* enthaltenes Fucoidan als ein Fucoidan mit VEGF-hemmender Eigenschaft. Weitere Fucoidane mit VEGF-hemmenden Eigenschaften sind in Algen der Gattung *Laminaria* enthalten, insbesondere *Laminaria digitata.* Weitere Fucoidane mit VEGF-hemmenden Eigenschaften können ebenso in Spezies des Taxons *Laminariales* und/oder *Fucales* enthalten sein.

Gemäß einem Aspekt umfasst die Zusammensetzung ein Mikronährstoff-Gemisch. Das Mikronährstoff-Gemisch kann einen oder mehr der folgenden Bestandteile umfassen: Vitamin C (Ascorbinsäure), Vitamin E (Tocopherol), Zink, Kupfer, Lutein und/oder Zeaxanthin. Das Mikronährstoff-Gemisch kann alle der genannten Bestandteile umfassen. Zusätzlich zu den genannten Bestandteilen kann das Mikronährstoff-Gemisch Docosahexaensäure und/oder Eicosapentaensäure, sowie eine Kombination daraus umfassen.

Die Bestandteile des Mikronährstoff-Gemisches können vorzugsweise in einer oral bioverfügbaren Form vorliegen. Insbesondere können die Bestandteile des Mikronährstoff-Gemisches in einer resorbierbaren Form vorliegen, beispielsweise in einer für das jeweilige Bestandteil üblichen Darreichungsform, wie sie in Nahrungsergänzungsmitteln oder zur oralen Aufnahme bestimmten Arzneimitteln bekannt ist. Die Bestandteile des Mikronährstoffgemisches können derivatisiert sein und/oder als Vorstufe vorliegen, wobei die Vorstufe beispielsweise im Organismus zum aktiven Bestandteil metabolisiert werden kann. Das Mikronährstoff-Gemisch kann ein bekanntes Mikronährstoffgemisch zur Verringerung des Fortschreitens einer AMD und/oder zur Verringerung der Wahrscheinlichkeit eines Sehverlusts bei AMD sein.

Beispielsweise kann Vitamin C als freie Säure oder als Ascorbat, beispielsweise Natrium-, Kalium- oder Calciumascorbat vorliegen. Beispielsweise kann Vitamin E sowohl als Vitamin, als auch als Provitamin, beispielsweise als ein Derivat eines Tocopherols, beispielsweise als Acetat, beispielsweise als α-Tocopherylacetat vorliegen. Beispielsweise kann Zink als Zn²⁺-Salz vorliegen, beispielsweise als Zinkoxid, Zinksulfat, Zink-Histidin, Zink-Aspartat oder Zinkorotat. Beispielsweise kann Kupfer als Cu⁺- oder Cu²⁺-Salz vorliegen, beispielsweise als Kupferoxid oder Kupfersulfat. Beispielsweise kann Lutein als freies Lutein oder als Luteinester vorliegen. Beispielsweise kann Zeaxanthin als freies Zeaxanthin oder als Zeaxanthinester vorliegen. Docosahexaensäure und/oder Eicosapentaensäure können als Bestandteile einer Fettsäuremischung vorliegen, wobei die Fettsäuremischung weitere Fettsäuren, insbesondere weitere Omega-3-Fettsäuren umfassen kann. Die Fettsäuren können als Ester oder Lipide, beispielsweise Phospholipide vorliegen.

Gemäß einem Aspekt wird eine Darreichungsform beschrieben. Die Darreichungsform kann zumindest eine Zusammensetzung gemäß einer hierin beschriebenen Ausführungsform oder einem hierin beschriebenen Aspekt umfassen. Die Darreichungsform kann für die orale Aufnahme vorgesehen sein. Beispielsweise kann die Darreichungsform eine feste Darreichungsform zur peroralen Anwendung sein. Beispielsweise kann die Darreichungsform eine Tablette, eine Kapsel, insbesondere eine Hart- oder Weichgelatinekapsel sein. Weitere Darreichungsformen wie beispielsweise Pulver, Granulat und/oder Liquid können vorgesehen sein, wobei die Darreichungsform Pulver, Granulat und/oder Liquid vorteilhafterweise als Sachet, Trinkampulle oder dergleichen bereitgestellt sein kann.

Gemäß einem Aspekt kann die Darreichungsform für die Gabe von Einzeldosen vorgesehen sein. Beispielsweise kann eine Tagesdosis vorgesehen sein, die durch die zeitversetzte Gabe von ein, zwei, drei, vier, oder sogar 5 oder mehr Einzeldosen, beispielsweise über den Tag verteilt, einnehmbar ist.

Gemäß einem Aspekt wird eine Darreichungsform oder Zusammensetzung gemäß einer hierin beschriebenen Ausführungsform oder einem hierin beschriebenen Aspekt zur Verwendung als Nahrungsergänzungsmittel beschrieben. Als Nahrungsergänzungsmittel kann insbesondere ein Nahrungsergänzungsmittel gemäß EU-Richtlinie 2002/46/EG und/oder der deutschen Nahrungsergänzungsmittelverordnung verstanden werden. Insbesondere können Bestandteile und Inhaltsstoffe der Darreichungsform oder Zusammensetzung in einer Menge und/oder Konzentration vorliegen, die eine Überdosierung oder Übersättigung zumindest bei gebrauchsgemäßer Verwendung vermeidet. Insbesondere können Bestandteile und Inhaltsstoffe der Darreichungsform in einer Menge und/oder Konzentration vorliegen, die eine Klassifikation der Darreichungsform oder Zusammensetzung als Arzneimittel nicht erfordert.

Gemäß einem Aspekt wird eine Darreichungsform oder Zusammensetzung gemäß einer hierin beschriebenen Ausführungsform oder einem hierin beschriebenen Aspekt zur Verwendung in einer ergänzenden bilanzierten Diät beschrieben. Bei der Darreichungsform oder Zusammensetzung kann es sich um diätetische Lebensmittel, insbesondere um ein diätetisches Lebensmittel für besondere medizinische Zwecke, beispielsweise gemäß der deutschen Verordnung über diätetische Lebensmittel handeln.

Gemäß einem Aspekt wird eine Darreichungsform oder Zusammensetzung gemäß einer hierin beschriebenen Ausführungsform oder einem hierin beschriebenen Aspekt zur Verwendung in der Behandlung und/oder Prävention von Erkrankungen der Netzhaut des Auges beschrieben. Bei der Erkrankung der Netzhaut des Auges kann es sich insbesondere um eine altersbedingte Makuladegeneration (AMD) handeln, insbesondere der feuchten Form der AMD. Als Prävention kann die Vermeidung der Erkrankung im gesunden Auge verstanden werden. Als Prävention kann die Vermeidung der Erkrankung im Auge, beispielsweise mit Indikatoren der zu erwartenden Erkrankung, oder beispielsweise eines Patienten mit bekannten Risikofaktoren, verstanden werden. Als Behandlung kann ein Vermeiden der Verschlechterung der Erkrankung verstanden werden. Als Behandlung kann eine Therapie, beispielsweise eine Symptomminderung oder Heilung der Erkrankung verstanden werden. Die Behandlung und/oder Prävention kann insbesondere beim Menschen, aber auch bei weiteren Säugetieren, beispielsweise Haus- und Hoftieren erfolgen. Bei der Darreichungsform oder Zusammensetzung kann es sich um eine Darreichungsform handeln.

Weitere Merkmale und Vorteile der vorliegenden Erfindung werden in der folgenden ausführlichen Beschreibung von bevorzugten Ausführungsformen der Zusammensetzung und/oder Darreichungsform vorgestellt. Auch wenn im Folgenden bevorzugte Ausführungsformen beschrieben werden, ist der Schutzbereich der Erfindung nicht auf dargestellte Ausführungsformen beschränkt, sondern umfasst auch ähnliche Ausführungsformen.

Gemäß einer ersten Ausführungsform umfasst eine erfindungsgemäße Zusammensetzung einen ersten Bestandteil, umfassend ein erstes Fucoidan, sowie einen zweiten Bestandteil, umfassend ein zweites Fucoidan. Der erste Bestandteil kann eine erste Algenspezies, beispielsweise die Alge *Saccharina latissima* umfassen. Der zweite Bestandteil kann eine zweite Algenspezies, beispielsweise die Alge *Laminaria hyperborea* umfassen. Sofern hierin auf die Algenspezies Bezug genommen wird, können darunter auch insbesondere Präparationen und/oder Extrakte der Algenspezies verstanden werden. Der erste Bestandteil und der zweite Bestandteil können beispielsweise getrocknete Pulver der jeweiligen Algen sein. In weiteren Ausführungsformen können Derivate, beispielsweise Fucoidan-haltige Extrakte, der jeweiligen Algen der erste und zweite Bestandteil sein. Das Fucoidan der Alge *Saccharina latissima* kann antioxidative Eigenschaften aufweisen. Das Fucoidan der Alge *Laminaria hyperborea* kann VEGF-hemmende Eigenschaften aufweisen.

In Ausführungsformen sind der erste Bestandteil und der zweite Bestandteil nicht besonders auf die Algenspezies beschränkt, sofern der erste Bestandteil ein Fucoidan umfasst, dessen antioxidative Eigenschaft mit der des Fucoidans von *Saccharina latissima* vergleichbar ist, und sofern der zweite Bestandteil ein Fucoidan umfasst, dessen VEGF-hemmende Eigenschaft mit der des Fucoidans von *Laminaria hyperborea* vergleichbar ist. Vergleichbare antioxidative Eigenschaften können dann gegeben sein, wenn eine vergleichbare Menge des Bestandteils, beispielsweise eine 0,5 bis 2-fache Menge des Bestandteils, in einem Antioxidations-Assay, beispielsweise einem Eisen-basierten Oxidations-Assay (z.B. FRAP), ein vergleichbares Ergebnis liefert. Vergleichbare VEGF-hemmende Eigenschaften können dann gegeben sein, wenn eine vergleichbare Menge des Bestandteils, beispielsweise eine 0,5 bis 2-fache Menge des Bestandteils, in einem Bindeassay, beispielsweise einem ELISA-Assay, ein vergleichbares Ergebnis liefert.

Vorteilhafterweise kann die Zusammensetzung der ersten Ausführungsform oral aufgenommen werden. Typischerweise basiert die Zusammensetzung auf einer ersten und einer zweiten Algenspezies, wobei die Algenspezies in der Regel als Nahrungsmittel bekannt sind. Somit kann die Zusammensetzung in vielen Fällen ohne das Risiko von unerwünschten oder unerwarteten Nebenwirkungen eingenommen werden und ist üblicherweise in einem hohen Dosisbereich gut verträglich. Eine Kombination aus einem Fucoidan mit antioxidativen Eigenschaften und einem Fucoidan mit VEGF-hemmenden Eigenschaften kann vorteilhafterweise die altersbedingte Makuladegeneration therapieren, vermeiden, abschwächen oder zumindest in ihrem Verlauf begünstigen. Somit kann die Zusammensetzung insbesondere als Nahrungsergänzungsmittel, diätetisches Lebensmittel oder Arzneimittel verwendbar sein.

Gemäß einer Ausführungsform kann die Zusammensetzung für die tägliche orale Aufnahme vorgesehen sein. In einer beispielhaften Ausführungsform kann für einen erwachsenen Menschen die folgende Zusammensetzung zur täglichen oralen Einnahme, beispielsweise über einen bestimmten Zeitraum, vorgesehen sein: *Laminaria hyperborea,* getrocknetes Pulver, 5 - 3000 mg, insbesondere 100 - 500 mg, insbesondere 150 - 400 mg, insbesondere 200 - 350 mg, beispielsweise 300 mg. Das getrocknete Pulver kann einen Gesamt-lod Anteil von 0,005 - 0,015 %, beispielsweise 0,008 - 0,012 %, beispielsweise 0,009 % aufweisen.

*Saccharina latissima,* getrocknetes Pulver, 5 - 3000 mg, insbesondere 30 - 300 mg, insbesondere 50 - 200 mg, insbesondere 75 - 150 mg, beispielsweise 100 mg. Das getrocknete Pulver kann einen Gesamt-lod Anteil von 0,02 - 0,18 %, beispielsweise 0,04 - 0,12 %, beispielsweise 0,061 % aufweisen.

Vorteilhafterweise kann der lod-Gehalt, insbesondere der Gesamt-Iod-Gehalt der Zusammensetzung, unterhalb einer Tageshöchstdosis an Iod liegen. Die Tageshöchstdosis an Iod kann einer täglichen Aufnahmeempfehlung, beispielsweise für Nahrungsergänzungsmittel, entsprechen. Beispielsweise kann die Tageshöchstdosis an Iod 500 µg, 200 µg, 150 µg, 100 µg oder sogar weniger als 100 µg betragen. Der Iod-Gehalt der Zusammensetzung kann beispielsweise dadurch angepasst werden, dass der Gesamt-Iod-Gehalt einer Charge z.B. des Algenpulvers bestimmt wird, und nur Chargen eingesetzt werden, für die in der Zusammensetzung eine Tageshöchstdosis an Iod nicht überschritten wird. Vorteilhaferweise kann eine Darreichungsform der Zusammensetzung eine Angabe über den Gesamt-Iod-Gehalt der Zusammensetzung enthalten.

In Ausführungsformen kann die Zusammensetzung Hilfsstoffe umfassen. Zu geeigneten Hilfsstoffen gehören beispielsweise Lactose, Dextrose, Succrose, Sorbitol, Manitol, Stärken, Akaziengummi, Calciumphosphat, Alginate, Traganth, Gelantine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon- Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H. P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Gemäß einer Weiterbildung der ersten Ausführungsform umfasst die Zusammensetzung zusätzlich ein Mikronährstoff-Gemisch. Bei dem Mikronährstoffgemisch kann es sich um ein Gemisch gemäß AREDS-Rezeptur (500 mg Vitamin C, 400 IE Vitamin E, 15 mg beta-Carotin, 80 mg Zink als Zinkoxid und 2 mg Kupfer als Kupferoxid) handeln. In nachteiliger Weise kann beta-Carotin jedoch in manchen Fällen eine Erhöhung des Krebsrisikos bewirken, sodass in vorteilhafter Weise das beta-Carotin durch eine Mischung aus Lutein (10 mg) und Zeaxanthin (2 mg) substituiert werden kann. Zusätzlich kann das Mikronährstoffgemisch Omega-3-Fettsäuren, beispielsweise Docosahexaensäure und/oder Eicosapentaensäure enthalten. Die Omega-3-Fettsäuren können eine Resorption von Lutein und Zeaxanthin verbessern.

In einer vorteilhaften Ausführungsform kann das Mikronährstoff-Gemisch zumindest einen der folgenden Bestandteile umfassen, oder eine beliebige Kombination der folgenden Bestandteile oder alle der folgenden Bestandteile umfassen. Die Mengen- bzw. Konzentrationsangaben können sich auf eine Tagesdosis für einen erwachsenen Menschen beziehen. Die Mengen- bzw. Konzentrationsangaben können sich auf eine Tagesdosis eines 75kg schweren, erwachsenen Menschen beziehen. Die Tagesdosis kann in Abhängigkeit des Gewichtes des Menschen anpassbar sein, insbesondere direkt proportional. Wie bereits einleitend erwähnt, können die Bestandteile als eine beliebige Kombination von bioverfügbaren Salzen, Derivaten, Provitaminen oder dergleichen vorliegen. Die Mengen- bzw. Konzentrationsangaben können sich auf den aktiven Bestandteil der Substanz beziehen:
Vitamin C: 50 - 1500 mg; Vitamin E: 20 - 300 mg (alpha-Tocopherol Äquivalent); Zink: 2-90 mg; Kupfer: 0,5-10 mg; Lutein: 0,5-20 mg; Zeaxanthin 0,1-20 mg.

Als weitere, optionale Bestandteile können Omega-3-Fettsäuren in einer Gesamtmenge von 50 - 1000 mg vorgesehen sein, davon beispielsweise 5 - 150 mg Eicosapentaensäure und 45 - 950 mg Docosahexaensäure.

In einem ersten Ausführungsbeispiel kann eine erfindungsgemäße Zusammensetzung die folgenden Bestandteile umfassen, wobei die Konzentrations- oder Mengenangaben sich auf eine Tagesdosis für einen erwachsenen Menschen beziehen können:
*Laminaria hyperborea:* getrocknetes Pulver, 300 mg, 0,009 % Gesamt-lod; *Saccharina latissima:* getrocknetes Pulver, 100 mg, 0,061 % Gesamt-lod; Vitamin C: 500 mg; Vitamin E: 90 mg (alpha-Tocopherol Äquivalent); Zink: 10 mg; Kupfer: 1 mg; Lutein: 10 mg; Zeaxanthin: 2 mg.

In einer Weiterbildung des ersten Ausführungsbeispiels kann die Zusammensetzung in einem zweiten Ausführungsbeispiel weiterhin umfassen:
Docosahexaensäure: 350 mg; Eicosapentaensäure: 650 mg.

In einer Weiterbildung des ersten oder zweiten Ausführungsbeispiels kann die Zusammensetzung in einem dritten Ausführungsbeispiel weiterhin Hilfsstoffe umfassen.

Für das hierin beschriebene Mikronährstoff-Gemisch ist bekannt, dass die Wahrscheinlichkeit eines Fortschreitens der AMD und die Wahrscheinlichkeit eines Sehverlustes signifikant gesenkt werden kann. Durch die Ergänzung der Zusammensetzung mit dem Mikronährstoff-Gemisch kann somit zusätzlich ein vorteilhafter Effekt erreicht werden, um die altersbedingte Makuladegeneration zu therapieren, zu vermeiden, abzuschwächen oder zumindest in ihrem Verlauf zu begünstigen. Der erste Bestandteil, der zweite Bestandteil und das Mikronährstoffgemisch können sich hierbei in überraschender und besonders vorteilhafter Weise ergänzen.

Gemäß einer Ausführungsform wird eine Darreichungsform beschrieben. Die Darreichungsform umfasst eine Zusammensetzung gemäß einer hierin beschriebenen Ausführungsform und ist zur peroralen Anwendung geeignet. Die erfindungsgemäße Darreichungsform kann eine feste, halbfeste oder flüssige Darreichungsform sein. Als feste Darreichungsform kann sie ausgewählt sein aus der Gruppe bestehend aus Tablette, Kapsel, Pulver oder Granulat. Als halb-feste Darreichungsform kann sie ausgewählt sein, aus der Gruppe, bestehend aus Creme, Emulsion und Gel. Als flüssige Darreichungsform liegt sie bevorzugt in Form einer wässrigen oder wässrig-organischen Lösung (Liquid) oder Suspension vor. Bevorzugt ist die wässrige oder wässrig-organische Lösung oder Suspension hyperton.

In einer Ausführungsform ist die Darreichungsform eine Tablette. In der Darreichungsform der Zusammensetzung der vorliegenden Erfindung kann ein Bindemittel, beispielsweise als Hilfsstoff, verwendet werden, um die physikalischen Eigenschaften von oralen Dosierungsformen, wie etwa daraus hergestellte Tabletten, zu verbessern. Wenn Zusammensetzung auch eine schlechte Verpressbarkeit (die Fähigkeit, in Tablettenform gepresst werden zu können) aufweist, ergeben solche Zusammensetzungen orale Darreichungsformen, die eine niedrige Festigkeit und eine inakzeptabel hohe Friabilität aufweisen. Die Zugabe eines Bindemittel-Materials zu solchen Zusammensetzungen verleiht der Darreichungsform Festigkeit und verbessert ihre physikalischen Eigenschaften. Beispiele für geeignete Bindemittel umfassen Stärken, Hydroxypropylmethylcellulose, Hydroxypropylcellulose, Ethylcellulose, Polyvinylpyrrolidon, Akazien-, Guargummi, Hydroxyethylcellulose, Agar, Calciumcarrageenan, Natriumalginat, Gelatine, Saccharide (einschließlich Glucose, Sucrose, Dextrose und Laktose), Molassen, Extrakt von Irisch Moos, Panwar-Gummi, Ghatti-Gummi, Muzilago aus Isapolschale ("isapol husk"), Carboxymethylcellulose, Methylcellulose, Veegum, Lärchen-Arbolaktan, Polyethylenglykole, Wachse und Mischungen davon.

In einer Ausführungsform ist die Darreichungsform eine Kapsel, wobei als Kapsel insbesondere auch Caplets, Gelkapseln, Cachets oder Pillen zu verstehen sind, die jeweils eine vorbestimmte Menge des aktiven Bestandteils als ein Pulver oder Granula enthalten, als eine Lösung oder eine Suspension in einer wässrigen Flüssigkeit oder einer nicht wässrigen Flüssigkeit, oder als eine Öl-in-Wasser-Flüssigkeitsemulsion oder eine Wasser-in-ÖI-Emulsion, etc. dargeboten werden.

In einer Ausführungsform ist die Darreichungsform ein Liquid. Das Liquid kann in flüssiger Form, beispielsweise zur direkten peroralen Aufnahme vorgesehen sein, oder zur Verdünnung in einer Flüssigkeit vorgesehen sein. Das Liquid kann durch die Lösung oder das in Suspension bringen eines Pulvers oder Granulats in einer Flüssigkeit herstellbar sein, wobei Liquid, Pulver oder Granulat beispielsweise in Sachets, beispielsweise als Einzelportionen oder -dosen verfügbar sein können.

In Ausführungsformen ist zum Erreichen einer Tagesdosis die Gabe von mehreren Einzeldosen vorgesehen. Die jeweilige Einzeldosis kann anteilig einen Teil der Stoffmenge der Tagesdosis der jeweiligen Bestandteile der Zusammensetzung beinhalten, beispielsweise kann für die Gabe von zwei Einzeldosen jede Einzeldosis die Hälfte der Tagesdosis des Bestandteils umfassen. Gleichermaßen kann für drei Einzeldosen jeweils ein Drittel, für vier Einzeldosen jeweils ein Viertel der Tagesdosis, oder für n Einzeldosen jeweils n⁻¹ der Tagesdosis vorgesehen sein. Eine jeweilige Einzeldosis kann sich, je nach vorgesehener Einnahmezeit, von den weiteren Einzeldosen unterscheiden, beispielsweise kann bei einer Tageserstdosis die Verabreichung einer höheren Dosis vorteilhaft sein, beispielsweise als Aufsättigungsdosis.

Gemäß einer weiteren Ausführungsform wird eine Zusammensetzung zur Verwendung bei der Behandlung und/oder Prävention der AMD bei Säugetieren beschrieben. Bei der Zusammensetzung kann es sich um eine Zusammensetzung gemäß hierin beschriebenen Ausführungsformen oder Aspekten handeln. Die Zusammensetzung kann als eine der hierin beschriebenen Darreichungsformen vorgesehen sein.

Die Ausführungsform der Zusammensetzung umfasst eine erste Braunalgenspezies und/oder Derivate davon, umfassend ein erstes Fucoidan, wobei das erste Fucoidan eine antioxidative Eigenschaft aufweist. Die Ausführungsform der Zusammensetzung umfasst eine zweite Braunalgenspezies und/oder Derivate davon, umfassend ein zweites Fucoidan, wobei das zweite Fucoidan eine VEGF-hemmende Eigenschaft aufweist. Die Ausführungsform der Zusammensetzung beinhaltet ein Mikronährstoff-Gemisch, umfassend Vitamin C, Vitamin E, Zink, Kupfer, Lutein, und Zeaxanthin als oral bioverfügbare Bestandteile.

Als Säugetiere sind insbesondere Menschen zu verstehen. Vorteilhafterweise ist die Zusammensetzung zur Behandlung und/oder Prävention der altersbedingten Makuladegeneration vorgesehen, sodass die Zusammensetzung insbesondere für Menschen ab dem 40., 50., 60. oder 70. Lebensjahr vorgesehen sein kann. Gleichermaßen kann die Zusammensetzung für weitere Säugetieren vorgesehen sein, beispielsweise Haustieren wie Caniden und Feliden, sowie Boviden, Cerviden, Equiden, Primaten oder sonstige Säugetiere. Hierbei ist naturgemäß in Abhängigkeit der typischen Lebenserwartung der Spezies eine Anpassung des entsprechenden Lebensalters, ab dem die Verwendung der Zusammensetzung besonders vorteilhaft sein kann, vorgesehen. Eine Darreichungsform der Zusammensetzung für nichtmenschliche Säugetiere kann beispielsweise als ein Pulver, Granulat, Liquid, oder dergleichen der üblichen Nahrung beigemischt werden.

Obwohl die vorliegende Erfindung vorstehend anhand typischer Ausführungsbeispiele beschrieben wurde, ist sie darauf nicht beschränkt, sondern auf vielfältige Weise modifizierbar. Auch ist die Erfindung nicht auf die genannten Anwendungsmöglichkeiten beschränkt.

## Patentansprüche

1. Zusammensetzung, umfassend:
- einen ersten Bestandteil, umfassend ein erstes Fucoidan;
- einen zweiten Bestandteil, umfassend ein zweites Fucoidan, wobei sich der erste Bestandteil vom zweiten Bestandteil unterscheidet.

2. Zusammensetzung nach Anspruch 1, wobei das erste Fucoidan eine antioxidative Eigenschaft aufweist, und/oder wobei das zweite Fucoidan eine VEGF-hemmende Eigenschaft aufweist.

3. Zusammensetzung nach Anspruch 1 oder 2, wobei der erste Bestandteil eine Algenspezies und/oder Derivate davon umfasst und/oder wobei der zweite Bestandteil eine Algenspezies und/oder Derivate davon umfasst.

4. Zusammensetzung nach einem der vorhergehenden Ansprüche, wobei der erste Bestandteil die Alge *Saccharina latissima* und/oder Derivate davon umfasst, und/oder wobei der zweite Bestandteil die Alge *Laminaria hyperborea* und/oder Derivate davon umfasst.

5. Zusammensetzung nach einem der vorhergehenden Ansprüche, weiterhin umfassend ein Mikronährstoff-Gemisch, wobei das Mikronährstoff-Gemisch zumindest einen der Bestandteile aus der Gruppe bestehend aus a.) bis f.) umfasst:
a.) Vitamin C;
b.) Vitamin E;
c.) Zink;
d.) Kupfer;
e.) Lutein; und
f.) Zeaxanthin.

6. Zusammensetzung nach Anspruch 5, des Weiteren umfassend
g.) Docosahexaensäure; und/oder
h.) Eicosapentaensäure.

7. Zusammensetzung nach einem der vorhergehenden Ansprüche 5 oder 6, wobei die Bestandteile des Mikronährstoff-Gemisches in einer oral bioverfügbaren Form vorliegen.

8. Darreichungsform, umfassend eine Zusammensetzung nach einem der vorhergehenden Ansprüche, wobei die Darreichungsform insbesondere eine Darreichungsform zur peroralen Anwendung umfasst, insbesondere Tablette, Kapsel Pulver, Granulat oder Liquid.

9. Darreichungsform oder Zusammensetzung nach einem der vorhergehenden Ansprüche zur Verwendung als Nahrungsergänzungsmittel, ergänzende bilanzierte Diät und/oder zur Verwendung in der Behandlung und/oder Prävention von Erkrankungen der Netzhaut des Auges, insbesondere altersbedingter Makuladegeneration (AMD), insbesondere beim Menschen.

10. Zusammensetzung zur Verwendung bei der Behandlung und/oder Prävention der altersbedingten Makuladegeneration (AMD) bei Säugetieren, umfassend:
eine erste Braunalgenspezies und/oder Derivate davon, umfassend ein erstes Fucoidan, wobei das erste Fucoidan eine antioxidative Eigenschaft aufweist;
eine zweite Braunalgenspezies und/oder Derivate davon, umfassend ein zweites Fucoidan, wobei das zweite Fucoidan eine VEGF-hemmende Eigenschaft aufweist;
ein Mikronährstoff-Gemisch, umfassend Vitamin C, Vitamin E, Zink, Kupfer, Lutein, und Zeaxanthin als oral bioverfügbare Bestandteile.

11. Verwendung der Zusammensetzung oder Darreichungsform nach einem der Ansprüche 1 bis 10 zur Behandlung und/oder Prävention der altersbedingten Makuladegeneration (AMD) bei Säugetieren.

12. Verwendung der Zusammensetzung oder Darreichungsform nach einem der Ansprüche 1 bis 10 zur Behandlung und/oder Prävention der altersbedingten Makuladegeneration (AMD) bei Menschen ab dem 40. Lebensjahr.
